# EUROPEAN PATENT SPECIFICATION

(11) **EP 1 322 158 B1**
(45) Date of publication and mention of the grant of the patent: **08.08.2012**
(21) Application number: 00960937.1
(22) Date of filing: 02.10.2000
(51) Int. Cl.: A01N 37/52, A61K 47/30, A61K 9/20, A61K 9/22, A61K 9/28

(54) **Sustained release pharmaceutical compositions containing metformin and method of their production**
Metformin enthaltende pharmazeutische Zusammensetzungen mit verzögerter Wirkstofffreigabe und Verfahren zu deren Herstellung
Compositions pharmaceutiques a liberation prolongee contenant de la metformine, procedes de production de ces dernieres

(43) Date of publication of application: 02.07.2003
(73) Proprietor: USV Ltd., Govandi, Mumbai 4000 88 (IN)
(72) Inventor: GIDWANI, Suresh, Kumar, Mumbai 400 088 (IN); SINGNURKAR, Purushottam, Mumbai 400 088 (IN); TEWARI, Prashant, Kumar, Mumbai 400 088 (IN)
(74) Representative: Beresford, Keith Denis Lewis
(86) International application number: PCT/IB2000/001404
(87) International publication number: WO 2002/028181

(56) References cited:
- WO-A-00/38655
- WO-A-96/08243
- WO-A-98/55107
- WO-A-99/47125
- WO-A1-99/47128
- US-A- 5 922 769
- US-A- 6 011 049
- US-A- 6 099 859
- US-A- 6 099 862
- US-A- 6 117 451
- ABDALLAH O Y: "Preparation and evaluation of metformin hydorchloride controlled release tablets" SCIENCES TECHNIQUES ET PRATIQUES STP PHARMA, EDITIONS DE SANTE, PARIS, FR, vol. 4, no. 1, 1988, pages 15-20, XP002128673 ISSN: 0758-6922

## Description

### Field of the Invention

The present invention relates to sustained release pharmaceutical preparations containing metformin hydrochloride which provides sustained release of metformin hydrochloride over a prolonged period of time and a method of producing it.

Metformin hydrochloride is a well known biguanide derivative (1,1-dimethylbiguanide monohydrochloride) which is widely used as oral antihyperglycemic agent in the management of noninsulin dependent diabetes mellitus (NIDDM).

Metformin hydrochloride being a highly water soluble drug (>300 mg/ml at 25°C), leads to the difficulty in making a sustained release dosage form.

Marketed preparations available earlier with 850 mg dose of metformin hydrochloride having label of retard tablets (Glucophase RTM retard) have not been able to demonstrate any advantage in limited volunteer trials. This is probably attributable to poor choice of polymers and low dosage, desired for sustained action.

US patent 5,955,106 by Moeckel, J. describes the process of making metformin hydrochloride 850 mg retard tablet containing hydrocolloid forming retarding agents and further control of release provided by film envelope. It however does not provide any justification for using 850 mg dose of metformin hydrochloride for delayed release preparation and the expected release rates from such compositions. This patent also does not give any in-vitro and in-vivo data to support its claims. Literature survey indicates metformin hydrochloride has only 40% to 60% bioavailability with high renal clearance. Hence the dose 850 mg may be insufficient to achieve therapeutic plasma concentration, around 1 µg/ml for a sufficient period of time and might require to take such tablets twice or thrice a day.

WO99/47128 by Timmins et al describes a biphasic controlled release delivery system for metformin hydrochloride with inner solid particulate phase and outer solid continuous phase utilizing hydrophilic and hydrophobic polymers. These tablets are hydrodynamically balanced and swell up to approximately three times their dry size following hydration However it is well documented that in supine position the tablet escapes through the pylorus of the stomach after administration, which may deteriorate the tablet's in-vivo performance. Also volume desired to maintain floating of the tablet is never enough in the stomach except in fed condition. Hence making such system is doubtful with reference to its performance. Another major limitation of this patent is about dosage of the metformin hydrochloride and formulation. For instance, examples cited provides formulation of 500 mg metformin hydrochloride with tablet weight of approximately 1.0 gm - Hence restricting to the use of low dose sustained release tablets of 500 mg or slightly more only and making it obligatory to take two tablets of 500 mg each time to provide sustained action.

The document "Preparation and evaluation of metformin hydrochloride controlled-release tablets" published in the journal "Sciences Techniques et Pratiques Pharmaceutiques" in 1988, vol. 4, no. 1, pages 15-20, by Abdallah et al. constitutes further relevant prior art.

The present invention is based on the scientific calculation of dose of metformin hydrochloride desired, based on the data available from in-vivo studies which are well documented in the scientific literature. The model used here is based on the mathematical equations provided by Dobrinska and Welling (1975) which give fairly accurate calculations about loading dose and maintenance dose for achieving sustained release effect.

The dose of metformin hydrochloride is calculated by considering the following pharmacokinetic values from the literature.
Plasma concentration Cmax = 1.02 µg/ml
Elimination half life t 1/2 = 6.2 hours.
Volume of distribution Vd = 275 litrs.
Renal clearance = 552± 139 Litrs/min.
Total clearance = 1300 ml/min.
Using Dobrinska and Welling model, the calculated loading dose is 283 mg and maintenance dose is 759 mg and the total dose is 1040 mg of metformin hydrochloride for achieving sustained release effect for 24 hours.

The object of the present invention is to prepare palatable and swallowable pharmaceutical preparation containing as high as approximately 1.0 gm metformin by suitable technology showing demonstrable release rate and facilitated in-vivo absorption for the desired period. The emphasis is to develop simple monolithic system composed of hydrophobic polymers and other excipients with improved kinetics of extended release dosage forms and with highest possible content of active substance and the simplest method of producing it.

In a first aspect, the present invention provides a monolithic sustained release pharmaceutical composition in tablet form comprising at least 74 % by weight of metformin hydrochloride based on the total weight of the composition, 15 to 40% of a hydrophobic polymer and/or other hydrophobic material based on the weight of metformin hydrochloride, the balance being auxiliary substances, wherein each tablet contains at least 1000mg metformin hydrochloride as the active substance.

In a second aspect, the present invention provides a process of producing a monolithic sustained release pharmaceutical composition as defined in claim 1 comprising:
(i) granulating metformin hydrochloride with hydrophobic polymer and/or other hydrophobic material by hot melt granulation to form a homogeneous granulate mass;
(ii) further granulating the mass with a binder solution and drying the granulated product;
(iii) compressing the dried granulated product into tablets each containing at least 1000 mg of metformin hydrochloride, and
(iv) optionally coating the tablets with a film envelope for taste neutralization.

In a third aspect, the present invention provides a process of producing a monolithic sustained release pharmaceutical composition as defined in claim 1 comprising:
(i) mixing metformin hydrochloride with hydrophobic polymer and/or other hydrophobic material and binder in an extruder to form a mixture;
(ii) extruding the mixture at a temperature of 40°C to 120°C to form melted homogeneous mass and cooling the melted mass;
(iii) milling and sizing the melted mass to form sized granules;
(iv) compressing the sized granules into tablets each containing at least 1000 mg of metformin hydrochloride, and
(iv) optionally coating the tablets with a film envelope for taste neutralization.

The monolithic sustained release system of the invention is a homogeneous system composed of active drug in an amount within the range of 60 to 90% by weight, preferably.70 to 80% by weight, and one or more hydrophobic polymers or one or more other type of hydrophobic materials. In an amount within the range of about 15 to 40% by weight, preferably 20 to 30 % by weight based on the weight of the active substance.

Hydrophobic polymers which may be employed for the monolithic sustained release system in the present invention include, but are not limited to stearic acid, glycerylmonostearate, glyceryl behenate, glyceryl monooleate, glyceryl palmitostearate, microcrystalline wax, stearyl alcohol, cetyl alcohol, cetostearyl alcohol, hydrogenated castor oil, tristearin, waxes, polyethylene powder, polyvinyl chloride, shellac, rosin, and the like. The mixtures of the hydrophobic polymer will be employed in weight ratio to other hydrophobic material within the range of about 1: 0.01 to 1: 5 , preferably about 1 : 0.3

The pharmaceutical compositions according to the present invention can be used to produce compressed tablets of any shape, preferably oval shape and can be additionally provided with a film coating for taste neutralization to which dye can optionally be abded for elegance. The proportion by weight of the film envelope relative to the final tablet is in the usual range of 0.5 to 4.0% by weight preferably 1.0 to 1.5% by weight Film formers such as hydroxypropyl methylcellulose, hydroxypropyl cellulose, starch, cellulose derivatives and the like can be used.

The monolithic composition according to the present invention can also be used to produce compressed slugs and filled into capsules.

Auxiliary substances employed for the monolithic sustained release system in the present invention include, binder, like polyvinyl pyrrolidone, gelatin, gum acacia, Klucel EF (hydroxypropyl cellulose), carboxymethyl cellulose sodium, etc.; glidants including but not limited to colloidal siliconedioxide, talc, starch, and the like; lubricants including but not limited to magnesium stearate, zinc stearate, and the like

The pharmaceutical dosage form according to the present invention such as tablet, apart from active drug and hydrophobic polymers and or hydrophobic materials may contain 1.0 to 15 % by weight of a binder, preferably 3.0 to 10 % by weight ; and upto 2.0 % by weight of glidant preferably 0.5 to 1.0 5 by weight; and upto 2.0 % by weight of lubricants preferably 0.5 to 1.0 % by weight ; each in relation to the tablet weight.

In the present invention the pharmaceutical composition, such as tablets are produced by dry mixing of active substance and optionally further auxiliary substance and granulating this mixture with hydrophobic polymers and or other hydrophobic materials by hot melt granulation technique using jacketed rapid mixer granulator at a temperature 40 to 120 °C, preferably 60 to 80 °C. This is followed by gradually cooling the granulate mass to the room temperature with continuous mixing. The resulting mass is further granulated with aqueous or organic solution of the binder followed by drying and converting it into 30 µm to 2.0 mm granules, preferably 100 µm to 1.0 mm by milling and sizing. Subsequently appropriate other pharmaceutical auxiliary substances are admixed with the sized granules.

In the present invention the pharmaceutical composition, such as tablets are also produced by dry mixing of active substance, optionally further auxiliary substances, hydrophobic polymers and or another hydrophobic materials and binder in an extruder. This mixture is extruded at a temperature of 40 to 120 °C, preferably 60 to 90 °C in a simple extruder used for injection molding of plastics, followed by extrusion of the melted homogeneous mass with gradual cooling to room temperature and converting into 30 µm to 2.0 mm granules, preferably 100 µm to 1.0 mm by milling and sizing. Subsequently appropriate other pharmaceutical auxiliary substances are admixed with the sized granules.

The composition produced in this manner is subsequently processed in the usual manner to produce pharmaceutical dosage forms, such as e.g. compressed into tablets or filling of pressed slugs into capsule. The tablets can be coated with a film using the standard coating processes and methods such as conventional coating pan or fluid coating process.

The sustained release tablets according to the present invention release metformin hydrochloride in a controlled manner which is suppose to provide an effect over a time period upto 24 hours, preferably over 18 hours as per the calculations.

Useful metformin sustained release formulations as per the invention show the following in-vitro drug release characteristics when tested in gastric fluid pH 1.2 for first hour and then in phosphate buffer pH 6.8 USP.

| **Time(Hrs)** | **% Release** |
|---|---|
| 1 | 38 - 45% |
| 2 | 50 - 55% |
| 3 | 62 - 68% |
| 4 | 70 - 75% |
| 5 | 80 - 85% |
| 6 | 85 - 90% |
| 7 | 91 - 95% |
| 8 | 96 - 100% |

### Example 1 :

225 gm of stearic acid was melted at 70°C temperature. 1000 gm metformin hydrochloride was heated to 70°C in a jacketed rapid mixer granulator and granulated with above melted stearic acid at 70°C temperature. After granulation the granulate mass was mixed continuously with gradual cooling to room temperature.

60 gm of shellac and 25 gm of polyvinyl pyrollidone were dissolved in 150 gm of isopropyl alcohol. This solution was gradually added to above metformin stearic acid granulate and mixed till dough mass formed. The resulting dough mass was dried at 45°C for 2 hours and then sized through 2.4 mm screen to break the agglomerates. These sized granules (1310 gm) were blended with 4.0 gm of colloidal silicone dioxide and 8.0 gm of magnesium stearate and compressed into capsule shape oval tablets each containing 1000 mg of metformin hydrochloride.

The in-vitro release of metformin hydrochloride from these tablets was as follows.

| **Time (Hrs)** | **% Release** |
|---|---|
| 1. | 40 % |
| 2 | 55 % |
| 3 | 65 % |
| 4 | 75 % |
| 5 | 82 % |
| 6 | 89 % |
| 7 | 95 % |
| 8 | 99.5 % |

### Example 2 :

225 gm of stearic acid , 1000 gm metformin hydrochloride, 60 gm of shellac and 25 gm of polyvinyl pyrollidone were mixed in the extruder at 70°C and extruded and then gradually cooled to room temperature. The resulting agglomerates were sized through 2.4 mm screen. These sized granules (1310 gm) were blended with 4.0 gm of colloidal silicone dioxide and 8.0 gm of magnesium stearate and compressed into capsule shape oval tablets each containing 1000 mg of metformin hydrochloride.

The in-vitro release of metformin hydrochloride from these tablets was as follows

| **Time (Hrs)** | **% Release** |
|---|---|
| 1 | 42% |
| 2 | 57% |
| 3 | 68% |
| 4 | 77% |
| 5 | 84% |
| 6 | 90% |
| 7 | 96% |
| 8 | 100% |

### Example 3 :

250 gm of glyceryl mono stearate was melted at 70°C temperature. 1000 gm metformin hydrochloride was heated to 70°C in a jacketed rapid mixer granulator and granulated with above melted stearic acid at 80°C temperature. After granulation the granulate mass was mixed continuously with gradual cooling to room temperature

60 gm of shellac and 25 gm of polyvinyl pyrollidone were dissolved in 150 gm of isopropyl alcohol. This solution was gradually added to above metformin stearic acid granulate and mixed till dough mass formed. The resulting dough mass was dried at 45°C for 2 hours and then sized through 2.4 mm screen to break the agglomerates. These sized granules (1335 gm) were blended with 4.0 gm of colloidal silicone dioxide and 8.0 gm of magnesium stearate and compressed into capsule shape oval tablets each containing 1000 mg of metformin hydrochloride.

The in-vitro release of metformin hydrochloride from these tablets was as follows.

| **Time (Hrs)** | **% Release** |
|---|---|
| 1 | 39% |
| 2 | 52 % |
| 3 | 61% |
| 4 | 72 % |
| 5 | 80% |
| 6 | 88% |
| 7 | 94% |
| 8 | 98 % |

### Example 4 :

175 gm of polyethylene powder, 1000 gm metformin hydrochloride and 25 gm of polyvinyl pyrollidone were mixed in the extruder at 70°C and extruded and then gradually cooled to room temperature. The resulting agglomerates were sized through 2.4 mm screen. These sized granules (1200 gm) were blended with 4.0 gm of colloidal silicone dioxide and 8.0 gm of magnesium stearate and compressed into capsule shape oval tablets each containing 1000 mg of metformin hydrochloride.

The in-vitro release of metformin hydrochloride from these tablets was as follows

| **Time (Hrs)** | **% Release** |
|---|---|
| 1 | 48 % |
| 2 | 54.2 % |
| 3 | 64 % |
| 4 | 73.4 % |
| 5 | 82 % |
| 6 | 90.3 % |
| 7 | 96 % |
| 8 | 99.7 % |

### Example 5 :

160 gm of polyvinyl chloride powder, 1000 gm metformin hydrochloride and 25 gm of polyvinyl pyrollidone were mixed in the extruder at 70°C and extruded and then gradually cooled to room temperature. The resulting agglomerates were sized through 2.4 mm screen. These sized granules (1185 gm) were blended with 4.0 gm of colloidal silicone dioxide and 8.0 gm of magnesium stearate and compressed into capsule shape oval tablets each containing 1000 mg of metformin hydrochloride.

The in-vitro release of metformin hydrochloride from these tablets was as follows.

| **Time (Hrs)** | **% Release** |
|---|---|
| 1 | 42 % |
| 2 | 53.1 % |
| 3 | 62.5 % |
| 4 | 72 % |
| 5 | 80% |
| 6 | 85 % |
| 7 | 94 % |
| 8 | 98.8 % |

### Example 6 :

230 gm of stearic acid melted at 70°C temperature. 1000 gm metformin hydrochloride was heated to 70°C in a jacketed rapid mixer granulator and granulated with above melted stearic acid at 70°C temperature. After granulation the granulate mass was mixed continuously with gradual cooling to room temperature.

60 gm of shellac and 25 gm of polyvinyl pyrollidone was dissolved in 150 gm of isopropyl alcohol. This solution was gradually added to above metformin stearic acid granulate and mixed till dough mass formed. The resulting dough mass was dried at 45°C for 2 hours and then sized through 2.4 mm screen to break the agglomerates. These sized granules (1315 gm) were blended with 4.0 gm of colloidal silicone dioxide and 8.0 gm of magnesium stearate and compressed into capsule shape oval tablets each containing 1000 mg of metformin hydrochloride.

The in-vitro release of metformin hydrochloride from these tables was as follows.

| **Time (Hrs)** | **% Release** |
|---|---|
| 1 | 41 % |
| 2 | 53 % |
| 3 | 66 % |
| 4 | 74.9 % |
| 5 | 83 % |
| 6 | 91 % |
| 7 | 96.2% |
| 8 | 100 % |

### References :

1. Moeckel, J., Gabel, R., Woog, H., [1997], U.S. Patent 5,955,106.
2. Timmins, P., Vyas, K., [1999], WO 99/47128.
3. Noel, M., (1980), Jounal of International Biomedical Information and Data (IBID), 1 (1), 9 - 20.
4. Kenneth, C., Ralph. A. D., (1998), Diabetes Reviews, 6 (2), 89 - 131.
5. Nancy C. Sambol, Jaine Chaing, Michael O'Conner, Chui Y. Liu, (1196), J. Clin. Pharmacol., 36, 1012 - 1021.
6. Physician Desk reference, Edition 58, (2000), Medical economic company Inc. NJ 07645-1742, Glucophage®, page 831-835.

## Claims

1. A monolithic sustained release pharmaceutical composition in tablet form comprising at least 74 % by weight of metformin hydrochloride based on the total weight of the composition, 15 to 40% of a hydrophobic polymer and/or other hydrophobic material based on the weight of metformin hydrochloride, the balance being auxiliary substances, wherein each tablet contains at least 1000mg metformin hydrochloride as the active substance.

2. The composition of claim 1, wherein the in-vitro drug release characteristics of said tablets when tested in gastric fluid of pH 1.2 for the first hour and then in phosphate buffer of pH 6.8 USP for the following 7 hours being as follows:
| Time (Hours) | % Release |
|---|---|
| 1 | 38-45 % |
| 2 | 50 - 55 % |
| 3 | 62-68 % |
| 4 | 70-75 % |
| 5 | 80 - 85 % |
| 6 | 85-90 % |
| 7 | 91-95% |
| 8 | 96-100% |

3. The composition of claim 1, wherein the hydrophobic polymer and/or hydrophobic material is selected from the group consisting of Fatty acids, Fatty alcohols, Fatty acid esters, Hydrogenated oils, waxes and natural resins.

4. The composition of claim 3, wherein the hydrophobic polymer and/or hydrophobic material is selected from the group consisting of stearic acid, glyceryl monostearate, glyceryl behenate, glyceryl palmitostearate, glyceryl monooleate, microcrystalline wax, stearyl alcohol, cetyl alcohol, cetostearyl alcohol, hydrogenated castor oil, tristearin, shellac, rosin, polyvinyl chloride powder and polyethylene powder.

5. Process of producing a monolithic sustained release pharmaceutical composition as defined in claim 1 comprising:
(i) granulating metformin hydrochloride with hydrophobic polymer and/or other hydrophobic material by hot melt granulation to form a homogeneous granulate mass;
(ii) further granulating the mass with a binder solution and drying the granulated product;
(iii) compressing the dried granulated product into tablets each containing at least 1000 mg of metformin hydrochloride; and
(iv) optionally coating the tablets with a film envelope for taste neutralization.

6. Process of producing a monolithic sustained release pharmaceutical composition as defined in claim 1 comprising:
(i) mixing metformin hydrochloride with hydrophobic polymer and/or other hydrophobic material and binder in an extruder to form a mixture;
(ii) extruding the mixture at a temperature of 40°C to 120°C to form melted homogeneous mass and cooling the melted mass;
(iii) milling and sizing of the melted mass to form sized granules;
(iv) compressing the sized granules into tablets each containing at least 1000 mg of metformin hydrochloride;
(v) optionally coating the tablets with a film envelope for taste neutralization.

7. The pharmaceutical composition of claim 1 for treating noninsulin dependent diabetes mellitus (NIDDM).

## Patentansprüche

1. Monolithische pharmazeutische Zusammensetzung mit verzögerter Wirkstofffreisetzung in Tablettenform, umfassend gewichtsmäßig mindestens 74 % Metformin-Hydrochlorid auf der Grundlage des Gesamtgewichts der Zusammensetzung, 15 bis 40 % eines hydrophoben Polymers und/oder eines anderen hydrophoben Materials auf der Grundlage des Gewichts des Metformin-Hydrochlorids, wobei der Rest Hilfssubstanzen sind, wobei jede Tablette mindestens 1000 mg Metformin-Hydrochlorid als die aktive Substanz enthält.

2. Zusammensetzung nach Anspruch 1, wobei die charakteristischen in-vitro-Wirkstofffreisetzungseigenschaften der Tabletten, wenn sie in Magensaft mit pH-Wert 1,2 während der ersten Stunde und dann in Phsophat-Sulfurlösung mit pH-Wert 6,8 USP während der folgenden 7 Stunden getestet werden, wie folgt aussehen:
| Zeit (Stunden) | % Freisetzung |
|---|---|
| 1 | 38-45 % |
| 2 | 50-55 % |
| 3 | 62-68 % |
| 4 | 70-75 % |
| 5 | 80-85 % |
| 6 | 85-90 % |
| 7 | 91-95 % |
| 8 | 96-100 % |

3. Zusammensetzung nach Anspruch 1, wobei das hydrophobe Polymer und/oder das hydrophobe Material ausgewählt wird aus der Gruppe, die aus Fettsäuren, Fettalkoholen, Fettsäureestern, hydrierten Ölen, Wachsen und natürlichen Harzen besteht.

4. Zusammensetzung nach Anspruch 3, wobei das hydrophobe Polymer und/oder das hydrophobe Material aus der Gruppe ausgewählt wird, die aus Stearinsäure, Glyceryl-Monostearat, Glyceryl-Behenat, Glyceryl-Palmitostearat, Glyceryl-Monooleat, Mikrokristallinwachs, Stearylalkohol, Cetylalkohol, Cetylstearylalkohol, hydriertem Rizinusöl, Tristearin, Schellack, Kolophonium, Polyvinylchloridpulver und Polyethylenpulver besteht.

5. Verfahren zum Herstellen einer monolithischen pharmazeutischen Zusammensetzung mit verzögerter Freisetzung wie in Anspruch 1 definiert, umfassend:
(i) Granulieren von Metformin-Hydrochlorid mit einem hydrophobem Polymer und/oder einem anderen hydrophoben Material durch Heißschmelzgranulierung, um eine homogene Granulatmasse zu bilden,
(ii) weiteres Granulieren der Masse mit einer Bindelösung und Trocknen des granulierten Ergebnisses;
(iii) Komprimieren des getrockneten granulierten Erzeugnisses in Tabletten, deren jede mindestens 1000 mg Metformin-Hydrochlorid enthält, und
(iv) wahlweise Ummanteln der Tabletten mit einer Filmhülle zur Geschmacksneutralisierung.

6. Verfahren zum Herstellen einer monolithischen pharmazeutischen Zusammensetzung mit verzögerter Freisetzung wie in Anspruch 1 definiert, umfassend
(i) Mischen von Metformin-Hydrochlorid mit einem hydrophoben Polymer und/oder einem anderen hydrophoben Material und einem Bindemittel in einer Extrudiervorrichtung, um eine Mischung zu bilden;
(ii) weiter Extrudieren der Mischung bei einer Temperatur von 40 °C bis 120 °C, um eine geschmolzene homogene Masse zu bilden, und Kühlen der geschmolzenen Masse;
(iii)Mahlen und größenmäßiges Sortieren der geschmolzenen Masse, um größenmäßig sortierte Körnchen zu bilden;
(iv) Komprimieren der größenmäßig sortierten Körnchen in Tabletten, deren jede mindestens 1000 mg Metformin-Hydrochlorid enthält;
(v) wahlweise Ummanteln der Tabletten mit einer Filmhülle zur Geschmacksneutralisierung.

7. Pharmazeutische Zusammensetzung nach Anspruch 1 zum Behandeln von nicht-Insulin-abhängigen Diabetes mellitus (NIDDM).

## Revendications

1. Composition pharmaceutique à libération prolongée monolithique sous forme de comprimé, comprenant au moins 74 % en poids de chlorhydrate de metformine sur la base du poids total de la composition, 15 à 40 % d'un polymère hydrophobe et/ou d'une autre matière hydrophobe sur la base du poids du chlorhydrate de metformine, le reste consistant en substances auxiliaires, chaque comprimé contenant au moins 1000 mg de chlorhydrate de metformine comme substance active.

2. Composition suivant la revendication 1, dans laquelle les caractéristiques de libération du médicament in vitro desdits comprimés, lors d'un test dans du fluide gastrique à pH 1,2 pendant la première heure et ensuite dans un tampon au phosphate à pH 6,8 USP pendant les 7 heures suivantes, sont les suivantes :
| Temps (heures) | % de libération |
|---|---|
| 1 | 38 - 45 % |
| 2 | 50 - 55 % |
| 3 | 62 - 68 % |
| 4 | 70 - 75 % |
| 5 | 80 - 85 % |
| 6 | 85 - 90 % |
| 7 | 91 - 95 % |
| 8 | 96 - 100 % |

3. Composition suivant la revendication 1, dans laquelle le polymère hydrophobe et/ou la matière hydrophobe est choisi dans le groupe consistant en des acides gras, des alcools gras, des esters d'acides gras, des huiles hydrogénées, des cires et des résines naturelles.

4. Composition suivant la revendication 3, dans laquelle le polymère hydrophobe et/ou la matière hydrophobe est choisi dans le groupe consistant en l'acide stéarique, le monostéarate de glycéryle, le béhénate de glycéryle, le palmitostéarate de glycéryle, le monooléate de glycéryle, une cire microcristalline, l'alcool stéarylique, l'alcool cétylique, l'alcool cétostéarylique, l'huile de ricin hydrogénée, la tristéarine, la gomme laque, la colophane, une poudre de poly(chlorure de vinyle) et une poudre de polyéthylène.

5. Procédé pour la production d'une composition pharmaceutique à libération prolongée monolithique telle que définie dans la revendication 1, comprenant :
(i) la granulation de chlorhydrate de metformine avec un polymère hydrophobe et/ou une autre matière hydrophobe par granulation en masse fondue à chaud pour former une masse granulée homogène ;
(ii) une granulation supplémentaire de la masse avec une solution de liant et le séchage du produit granulé ;
(iii) la compression du produit granulé séché en comprimés contenant chacun au moins 1000 mg de chlorhydrate de metformine ; et
(iv) facultativement, l'enrobage des comprimés avec une enveloppe formant un film pour la neutralisation du goût.

6. Procédé pour la production d'une composition pharmaceutique à libération prolongée monolithique telle que définie dans la revendication 1, comprenant :
(i) le mélange de chlorhydrate de metformine avec un polymère hydrophobe et/ou une autre matière hydrophobe et un liant dans une extrudeuse pour former un mélange ;
(ii) l'extrusion du mélange à une température de 40°C à 120°C pour former une masse homogène fondue et le refroidissement de la masse fondue ;
(iii) le broyage et le calibrage de la masse fondue pour former des granules calibrés ;
(iv) la compression des granules calibrés en comprimés contenant chacun au moins 1000 mg de chlorhydrate de metformine ;
(vi) facultativement, l'enrobage des comprimés avec une enveloppe formant un film pour la neutralisation du goût.

7. Composition pharmaceutique suivant la revendication 1, pour le traitement du diabète sucré non insulino-dépendant (NIDDM).
